# EUROPEAN PATENT APPLICATION

(11) **EP 4 498 382 A1**
(43) Date of publication of application: **29.01.2025**
(21) Application number: 23187207.8
(22) Date of filing: 24.07.2023
(51) Int. Cl.: G16H 40/60, G16H 20/70, G16H 50/70, A61B 5/055, A61B 5/16, A61B 6/03

(54) **AMBIENT EXPERIENCE CONTROL DEVICE AND METHOD**

(71) Applicant: Koninklijke Philips N.V., 5656 AG Eindhoven (NL)
(72) Inventor: VAN EE, Raymond, 5656AG Eindhoven (NL); SPELT, Hanne Adriana Alijda, 5656AG Eindhoven (NL); BULUT, Murtaza, 5656AG Eindhoven (NL); BEREZHNOY, Igor, 5656AG Eindhoven (NL); KLAASSEN, Remy, 5656AG Eindhoven (NL)
(74) Representative: Philips Intellectual Property & Standards

(57) **Abstract**

The present invention relates to a device, system and method for controlling the delivery of a personalized ambient experience to a user during a medical imaging scan. The device comprises an input unit that obtains scan information and user information. A processing unit predicts the future user state based on the obtained scan information and the obtained user information, determines if the predicted future user state may cause a disruption of the medical imaging scan, and generates, if it has been determined that the predicted future user state may cause a disruption of the medical imaging scan, one or more ambient experience control signals configured to control one or more ambient experience units to generate or change a respective ambient experience provided by the respective ambient experience device. An output unit outputs the one or more ambient experience control signals to the one or more ambient experience units.

## Description

### FIELD OF THE INVENTION

The present invention relates to an ambient experience control device and method for controlling the delivery of a personalized ambient experience to a user during a medical imaging scan. The present invention relates further to a system for providing a personalized ambient experience to a user during a medical imaging scan.

### BACKGROUND OF THE INVENTION

Interaction with the patients in the bore of a medical imaging scanner (e.g. a CT or MR scanner) is generally accomplished via sound/intercom or a screen (e.g., breath holds). Sometimes there is an unplanned interaction, for example if the technologist needs to adjust or put someone at ease. These interactions are obtrusive to the scanning procedure and experience of the staff and patient. They take time and often come too late, and the scan may already be interrupted. In addition, it is known that patients (herein, more generally, called users) show better (in particular, less noisy and less variable) data, leading to less costs, when they feel engaged and have ownership by knowing what will happen next.

Other medical imaging procedures, such as X-ray or ultrasound imaging procedures, even if the user is positioned in a more open environment and not in a narrow bore, may suffer from disruptions caused by the user as well, particularly if the user shall remain in a certain position for a longer duration. Further, disruptions may appear in medical treatment procedures, such as radiotherapy procedures.

US 2013/211236 A1 discloses systems, devices and methods for performing a magnetic resonance imaging (MRI) scan of a patient. A method of performing an MRI scan on a patient can include monitoring a physiological signal level of the patient, analyzing the monitored physiological signal level, and providing instructions to the patient and/or changing the environmental conditions exposed to the patient based on the monitored physiological signal level. The instructions can include an acoustic command and/or a visual command. The changing of the environmental conditions can include visual simulation, acoustic stimulation and/or air conditioning change. The physiological signal level may comprise signal levels regarding breathing and/or body motion and/or cardiac activity of the patient.

### SUMMARY OF THE INVENTION

It is an object of the present invention to enable to perform a medical procedure, such as a medical imaging scan and a medical treatment procedure, with fewer or no disruptions caused by the user.

In a first aspect of the present invention an ambient experience control device for controlling the delivery of a personalized ambient experience to a user during a medical imaging scan is presented, the device comprising:
- - an input unit configured to obtain
   - scan information indicating scan-related information of a medical imaging scan including scan type information indicating the type of medical imaging scan and
   - user information indicating user-related information including user state information indicating the current user state during the medical imaging scan;
- - a processing unit configured to
   - predict the future user state based on the obtained scan information and the obtained user information,
   - determine if the predicted future user state may cause a disruption of the medical imaging scan, and
   - generate, if it has been determined that the predicted future user state may cause a disruption of the medical imaging scan, one or more ambient experience control signals configured to control one or more ambient experience units to generate or change a respective ambient experience provided by the respective ambient experience device; and
- an output unit configured to output the one or more ambient experience control signals to the one or more ambient experience units.

In a further aspect of the present invention an ambient experience control system for proving a personalized ambient experience to a user during a medical imaging scan is presented, the system comprising:
- a medical imaging scanner configured to perform a medical imaging scan of a user;
- one or more user state acquisition units configured to acquire user state information indicating the current user state during the medical imaging scan;
- an ambient experience control device as disclosed herein for controlling the delivery of a personalized ambient experience to a user during the medical imaging scan; and
- one or more ambient experience units configured to provide an ambient experience to the user according to one or more ambient experience control signals generated by the one or more ambient experience control units.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to anticipate or predict the user state so that a new event can be created to interact with or influence the user, to generate or modify an ambient experience (AE) event provided to the user in a personalized form. The AE event may be any sensory event provided to the user, e.g., as communication via sound, screen, other sensory modalities that may cause a desired change of the user state or avoid an undesired user state. This may prevent disruptive events, improve user experience and engagement by providing a personalized experience, improve staff experience by preventing disrupting events resulting in time saving, and influence user state unobtrusively (i.e., without the user realizing it) by adapting, e.g., light and sound and without any effort of the staff needed.

In contrast to the present invention, the systems, devices and methods disclosed in US 2013/211236 A1 do not predict the future user state, but recognize motion of the user, which may already have caused a disruption and then changes the AE. Further, according to the present invention, scan information indicating scan-related information of a medical imaging scan including scan type information indicating the type of medical imaging scan is used to predict the future user state and find out if the predicted user state may cause disruptions so that it may be decided to change the AE to bring the user into a more optimal user state. For instance, in an embodiment, an optimal user state may be determined, which may then be compared to a monitored physiological signal level of the user to decide if and which change of the AE should be made. Different scan types (and scanners) may require different optimal user states. Further, in case of multiple inputs (such as cardiovascular, electrodermal, respiratory, muscular, facial expression, etc.), certain input parameters may be prioritized over other input parameters depending on scan type or exam card, as provided according to another embodiment.

In this context, the expression "user state" shall be understood as physiological and/or psychological state of the user in terms of one or more parameters derived from the user related to the user's physiological and/or psychological state. For example, user heart rate, respiration rate, skin temperature, skin conductance response, speech, motions etc. can be used to determine the user state. User state may thus refer to a region in a multi-dimensional coordinate system, where different axes correspond to different user related measurements that are available, such as different parameters that can be extracted from user signals. User signal may be heart activity signal, respiration signal, speech signal, or motion signal (e.g., heart rate, respiration rate, speech energy, speech pitch, etc.). In an embodiment, heart rate and respiration rate may be sufficient to determine the user state.

Generally, "states" are specific patterns in these signals, often in a specific context for a certain time. They may differ from reactivity, which is a short instantaneous response to something, and levels or traits, which are more long lasting and have less variation between situations/contexts. Thereby, user state also concerns pattern analyses and sometimes an interpretation of the aforementioned parameters.

A "user" may be a "patient" or any other "subject" undergoing a medical imaging scan.

The expression "ambient experience" (sometimes also called "ambient user experience" or "patient perception content" or "user perception content") shall be understood as the experience of the user (patient) of or related to the user's surroundings, i.e., influences of the surroundings onto any of the user's senses. The same or similar effects may be achieved for clinical staff. It relates to one or more elements of the space design, e.g. the clinical space design, which aims to make the experience more pleasant for users/patients and staff. Elements of ambient experience may include one or more of dynamic lighting, projections and sounds as positive distractions for patients. Providing a pleasant ambient experience contributes to a motivating environment that has a positive impact on the quality of care.

According to an embodiment of the disclosed device, the processing unit is configured to determine a trend of the user state over a period of time based on multiple data points of one or more pieces of information of the obtained scan information and the obtained user information and to predict the future user state based on the determined trend. This provides a simple but efficient solution to determine the future user state.

Another way to predict the user state is to train a machine learning (ML) algorithm or neural network, where training data are profile (demographics), medical data, physiological data from past scans, scan data from previous scans, and, if available, ambient experience data. If enough data is collected from the medical imaging system with the AE, then this data can be used to train machine learning or any other artificial intelligence (AI) algorithms to predict the future user state. The ML can work so that the user state can be predicted sufficiently ahead (e.g., 5-30 minutes ahead) allowing sufficient time for AE to influence the user.

According to another embodiment the processing unit is configured to determine whether the predicted future user state is in line with a predetermined user state, in particular a predetermined user state for the type of medical imaging scan. For each scan or type of scan (or even each type of scanner) a particular user state may be desired or predetermined as optimal or minimal (i.e., a state that should at least be obtained). This desired or optimal or minimal user state may be compared to the predicted future user state to determine if any changes are preferred. For instance, the processing unit may be configured to determine whether the predicted future user state is in line with a predetermined user state by determining if the predicted future user state is below an upper state threshold or above a lower state threshold. The thresholds may be predetermined for the particular scan type and/or type of scanner and may relate to one or more parameters for determining the user state or to the user state as such, e.g. a score or indicator rating the user state on a particular scale (e.g. between 1 and 10). In an exemplary implementation, a distance metric (or measure) may be calculated between the predicted and required user state signals. Then the distance metric features (the mean/median value, distribution) may be used to determine how to drive the AE system, i.e., it may be checked if the corresponding distance is below the threshold. Cosine, Euclidian, and correlation are some example distance measures that can be employed.

The processing unit may further be configured to determine a disruption likelihood indicating the likelihood that the predicted future user state may cause a disruption of the medical imaging scan, and to generate, if the determined disruption likelihood is above a likelihood threshold, one or more ambient experience control signals, in particular adapt the ambient experience control signals depending on the disruption likelihood and/or the time and/or duration of a disruption. The disruption likelihood may e.g. be determined by use of Bayesian statistics.

Many different pieces of information may be obtained (i.e., received or retrieved) as input. The input unit (e.g. a data interface) may thus be configured to obtain, as scan information, information regarding one or more of type of medical imaging scan, length of medical imaging scan, type of medical imaging scanner, size of bore of the medical imaging scanner, sounds generated during medical imaging scan, and ideal user state during medical imaging scan. Further, the input unit may be configured to obtain, as user information, information regarding one or more of general user physiology, current user physiology, voice, movement, pressure, muscle activity, temperature, personality type, physiological trace before the medical imaging scan, health state, medication, scan history, problems during a prior medical imaging scan, needs reported by the user, frequency of problems during a medical imaging scan, eye movements, skin conductance level, sympathetic physiological arousal, stress level, pain level, electrodermal activity, sweating level, hand grip, respiration, pulse, heart rate variability, oxygen saturation, blood pressure, skin temperature, movement of one or more body parts, brain activity, user interaction information, user attention score, and game score. Hence, one or more of the following categories of data may thus be considered:
- historical data, demographics, etc.;
- data from past experiences;
- real-time measured physiological parameters, including movement; and
- real-time user engagement parameters (with a user interface (UI) or a technician or other person).

In this context it shall be noted that certain pieces of information can be both an input information and the predicted future user state. For instance, sympathetic arousal can be used as input (i.e. user state information) and/or can represent the predicted future user state.

Physiology can be one or more of cardiovascular activity (e.g. heart rate, heart rate variability, blood pressure), respiratory activity (e.g. breathing rate, breath depth), electrodermal activity (e.g. skin conductance peaks, skin conductance levels), muscle activity (in face and/or body), temperature, eye movements, and oxygen saturation. These input parameters are all subject to sympathetic and parasympathetic arousal.

Psychology can be one or more of personality type, psychological state, anxiety level, and reported needs.

Personal medical information can be one or more of age, gender, anamnesis, medication, scan history, and frequency of problems during a medical imaging scan.

There are various methods available to assess physiology and psychology continuously during a scan, which make them appropriate to assess momentary user state and adapt the environment. Personal medical information is static and may help with pre-selecting content or other parameters influencing AE or personalizing thresholds.

The processing unit may further be configured to generate one or more ambient experience control signals configured to control one or more of
- intensity, duration and/or color of light or images,
- intensity, duration, frequency, content and/or type of sound or voice,
- ambient temperature and/or heating temperature,
- intensity, duration and/or type of a tactile stimulation, and
- intensity, duration and/or type of an odor emission.

There may be guidelines or a look-up table available indicating which parameters to change in which direction to achieve a certain change of user state. These guidelines and this look-up table may be generated and updated over time based on the achieved results of effected changes of AE and the observed reactions of the user.

In another embodiment the input unit is configured to receive user feedback during the medical imaging scan and the processing unit is configured to use the received user feedback for generating the one or more ambient experience control signals. This may further improve the control of the AE and its effect on the user state.

The processing unit may further be configured to predict a time window when the user is going to move during the medical imaging scan based on one or more of
- movements of the user during a prior medical imaging scan;
- movements of the user during the medical imaging scan and/or during its preparation; and
- a database of typical movements and time windows of users as well as respective user information during a prior medical imaging scan. This may further improve the prediction of the future user state and the control of the generation or change of the AE.

The processing unit may further be configured to determine one or more of
- a general acceptable motion specific threshold indicating intensity, duration, type and frequency of motion that is generally acceptable and not regarded as causing a disruption of the medical imaging scan,
- a personalized acceptable motion specific threshold indicating intensity, duration, type and frequency of motion that is acceptable for the user and not regarded as causing a disruption of the medical imaging scan by the user,
- timing and/or type of motion-related disruption, and
- timing and/or type of motion of the user.

This information can additionally be used for the control of the AE to further improve the desired effect on the user state.

The proposed system for proving a personalized ambient experience to a user during a medical imaging scan comprises a medical imaging scanner configured to perform a medical imaging scan of a user, which may preferably be an MR, CT or X-ray imaging scanner.

The system further comprises one or more user state acquisition units configured to acquire user state information indicating the current user state during the medical imaging scan, which may comprise one or more of a user interface for inputting information, a retrieval unit for retrieving information from a database or record, a microphone, a movement detection unit, a pressure detection unit, a muscle activity detection unit, a temperature measurement unit, an eye tracking unit, a skin conductance measurement unit, a sympathetic physiological arousal detection unit, a stress level detection unit, a pain level detection unit, an electrodermal activity detection unit, a sweating level detection unit, a hand grip detection unit, a respiration sensor, a pulse sensor, a heart rate variability detection unit, an oxygen saturation sensor, and a blood pressure sensor.

The system further comprises an ambient experience control device as described above for controlling the delivery of a personalized ambient experience to a user during the medical imaging scan.

Still further, the system may comprise one or more ambient experience units configured to provide an ambient experience to the user according to one or more ambient experience control signals generated by the one or more ambient experience control units, for instance, one or more of a stimulation unit configured to stimulate one or more user senses, a stimulating environment combined with scan information, a stimulating environment where technician input and/or involvement is possible, a display, a lighting unit, a loudspeaker, an air condition, a body heating element and an odor dispenser.

Optionally, the system may further comprise a user interface allowing a user to provide user feedback during the medical imaging scan, in particular a button, voice analysis unit, or eye tracking unit.

### BRIEF DESCRIPTION OF THE DRAWINGS

These and other aspects of the invention will be apparent from and elucidated with reference to the embodiment(s) described hereinafter. In the following drawings.
Fig. 1 shows a schematic diagram of an embodiment of a system for proving a personalized ambient experience according to the present invention;
Fig. 2 shows a schematic diagram of an embodiment of an ambient experience control device for controlling the delivery of a personalized ambient experience according to the present invention;
Fig. 3 shows a flowchart of an embodiment of an ambient experience control method according to the present invention;
Fig. 4 shows diagrams of a sympathetic arousal signal, a predicted sympathetic arousal signal and an AE energy signal; and
Fig. 5 shows a flowchart of another embodiment of an ambient experience control method according to the present invention.

### DETAILED DESCRIPTION OF EMBODIMENTS

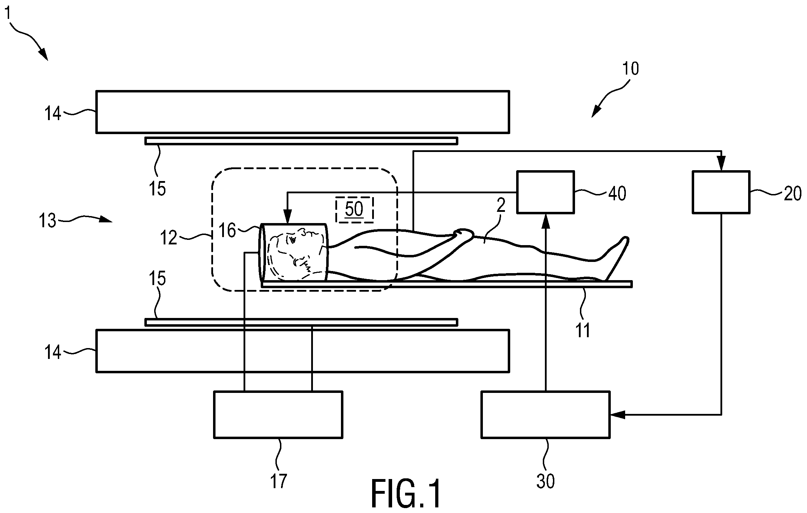

Fig. 1 schematically shows an embodiment of a system 1 for proving a personalized ambient experience to a user (e.g. a patient or other subject) 2 during a medical imaging scan. The system 1 comprises a medical imaging scanner 10, one or more user state acquisition units 20, an ambient experience control device 30 and one or more ambient experience units 40.

The medical imaging scanner 10 is configured to perform a medical imaging scan of a user. The medical imaging scanner 10 in this embodiment is a magnetic resonance imaging (MRI) scanner, but may generally be any other medical imaging scanner, such as a computed tomography (CT) scanner, positron emission tomography (PET) scanner, etc. During the medical imaging scan the user 2 is usually lying on a patient table 11, which at least part of the body placed in an imaging area 12 and inside a bore 13 surrounded by parts of the scanner (e.g. various coils 14, 15, 16 in case of an MRI scanner or a ring on which an X-ray source and/or an X-ray detector are rotating around the user). In other embodiments of a medical imaging scanner, an X-ray source and detector are fixed at opposite ends of an arc (e.g. a C-arc) and rotate around the user. For controlling the medical imaging scanner 10 and evaluating the acquired measurement signals a computer system 17 or processor or other circuitry is used, as is generally known and shall not be explained in more detail here.

The one or more user state acquisition units 20 are configured to acquire user state information indicating the current user state during the medical imaging scan. The one or more user state acquisition units 20 may comprise one or more of a user interface for inputting information, a retrieval unit for retrieving information from a database or record, a microphone, a movement detection unit, a pressure detection unit, a muscle activity detection unit, a temperature measurement unit, an eye tracking unit, a skin conductance measurement unit, a sympathetic physiological arousal detection unit, a stress level detection unit, a pain level detection unit, an electrodermal activity detection unit, a sweating level detection unit, a hand grip detection unit, a respiration sensor, a pulse sensor, a heart rate variability detection unit, an oxygen saturation sensor, and a blood pressure sensor.

The ambient experience control device 30 is configured to control the delivery of a personalized ambient experience to a user during the medical imaging scan. The ambient experience control device 30 may be implemented as computer, processor or other circuitry that obtains the user state information acquired by the one or more user state acquisition units 20 and scan information indicating scan-related information of the medical imaging scan including scan type information indicating the type of medical imaging scan, which may be provided by the medical imaging scanner 10 (e.g. the computer system 17) and/or may be inputted by an operator of the medical imaging scanner 10. An embodiment of the ambient experience control device 30 will be explained in more detail below.

The one or more ambient experience units 40 are configured to provide an ambient experience to the user according to one or more ambient experience control signals generated by the ambient experience control device 30. The one or more ambient experience units 40 may comprise one or more of a display, a lighting unit, a loudspeaker, an air condition, a body heating element and an odor dispenser.

The ambient experience units 40 may further comprise one or more of a stimulation environment/input (stimulating one or more user senses), a stimulating environment combined with scan information (where real-time data linked to scan progress may be used together with external stimuli), and a stimulating environment where technician input/involvement is possible. Stimulation may be visual stimulation (e.g., by use of a screen or projection on a surface, using video, or colored light, or a range of light brightness), and/or auditory stimulation (e.g. by tones, or a song, or a voice, etc.) and/or a stimulation of any other human sense. Further, the AE may include real-time scan data and/or clinician/technician involvement in addition to the sensory stimulators. To include scan data may e.g. mean that based upon a current real-time scan result (e.g. it becomes clear that a patient has a damaged auditory cortex) the sensory stimulation content becomes adapted based upon this new knowledge.

Generally, the one or more ambient experience units 40 may be understood as sensory stimulus source to provide a sensory stimulus, an entertainment feed and/or information to the user 2 during the medical imaging operation. Such sensory stimuli may include a signal that can be provided to the subject via any of (or combination of) the physical senses of the body, such as touch, smell, taste, hearing and sight. The sensory stimulus source may include a light or display to present a visual stimulus to the subject while positioned in the imaging region (including possibly a projection screen and/or optical system to relay displayed information to within the bore volume), and/or a speaker or sound source to present an auditory stimulus to the subject (including possibly a relay system, e.g. using guided air pressure transmission or contact conduction of sound into the bore volume, e.g. to a headphone). The sensory stimulus source can be used to provide entertainment to the user, to inform the user about the progress of the imaging procedure being performed and/or to intentionally trigger activity in a brain region of interest in the user (e.g. in a functional MRI examination).

The system 1 may optionally further comprise a user interface 50 that allows a user to provide user feedback during the medical imaging scan. The user interface 50 may, e.g., include a button, a voice analysis (or recognition) unit, and/or an eye tracking unit. Generally, other means allowing a user to provide feedback may be used as well. One or more of the user state acquisition units 20 may optionally be used as user interface as well.

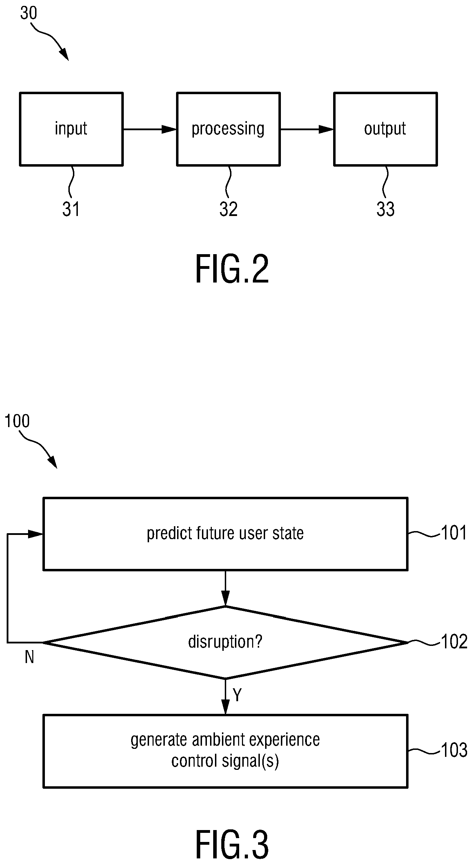

Fig. 2 schematically shows an embodiment of the ambient experience control device 30 for controlling the delivery of a personalized ambient experience (i.e., a personalized user perception content) to a user during a medical imaging scan. A flowchart of a corresponding ambient experience control method 100 is shown in Fig. 3. The device 30 comprise an input unit 31, a processing unit 32 and an output unit 33.

The input unit 31 is configured to obtain scan information indicating scan-related information of a medical imaging scan including scan type information indicating the type of medical imaging scan and user information indicating user-related information including user state information indicating the current user state during the medical imaging scan. The input unit 31 may be directly coupled or connected to medical imaging scanner 10 (e.g. the computing system 17) and/or the one or more user state acquisition units 20. Alternatively or additionally, the input unit 31 may be connected to a storage, buffer, network, or bus, etc. to receive or retrieve the required pieces of information. The input unit 21 may thus e.g. be (wired or wireless) communication interface or data interface, such as a Bluetooth interface, Wi-Fi interface, LAN interface, HDMI interface, direct cable connect, or any other suitable interface allowing signal transfer to the device 30.

The processing unit 32 is configured to carry out the steps of the ambient experience control method 100. In a first step 101 it predicts the future user state based on the obtained scan information and the obtained user information. In a second step 102 it determines if the predicted future user state may cause a disruption of the medical imaging scan. In a third step 103 it generates, if it has been determined that the predicted future user state may cause a disruption of the medical imaging scan, one or more ambient experience control signals configured to control one or more ambient experience units to generate or change a respective ambient experience provided by the respective ambient experience device. Otherwise, the operation continues with step 101.

The processing unit 32 may be any kind of means configured to process the obtained scan information and the obtained user information and generate one or more ambient experience control signals. It may be implemented in software and/or hardware, e.g. as a programmed processor or computer or other processing circuitry.

The output unit 33 is configured to output the one or more ambient experience control signals to the one or more ambient experience units. The output unit 33 may generally be any interface that provides the determined the one or more ambient experience control signals to the one or more ambient experience units 40, e.g., transmits them to the one or more ambient experience units 40. It may thus generally be any (wired or wireless) communication or data interface.

The present invention thus provides a novel way to present personalized ambient experience based on predicted state of the user for enhanced engagement using optimal user state for scan type as to prevent disruptive events. Different types of inputs can be used, in particular user state (e.g., physiology, voice analysis, movement, pressure, muscle activity, temperature) and scan type (incl. ideal user state, length, and sounds). Optional inputs include user traits (e.g., personality type or physiological trace of the user before the scan), exam card information and data-based information on how likely it is that the user may be in an undesired state during a particular sequence, which then may lead e.g. to motion.

In an embodiment, based on several data points of the input signal(s) representing user state (e.g., collected over one or more (e.g., 3+) minutes) a trend in user state may calculated, which may then be used to predict the future user state. It may be assessed whether the predicted future user state is in line with an ideal user state for that specific scan and the likelihood of disruption may be predicted. Based on this information, the AE content (and optionally communication content) plan may be updated to influence the user state towards an optimal state for that specific scan. The content may be updated on an AE system or any other form of user interface.

In a practical embodiment, the exam type (including ideal user state, length, and sounds) and the continuous skin conductance level (SCL) / skin conductance response (SCR) are used as input signals. Sympathetic physiological arousal can be used as indicators of pain or stress (as e.g. described by J. T. Cacioppo, L. G. Tassinary, and G. G. Berntson, The handbook of psychophysiology, 3rd ed., vol. 44. New York: Cambridge University Press, 2007, p. 511). Especially, electrodermal activity, i.e., sweating, has been linked to pain experiences, e.g. by H. Storm, "Changes in skin conductance as a tool to monitor nociceptive stimulation and pain," Curr. Opin. Anaesthesiol., vol. 21, no. 6, pp. 796-804, 2008. Furthermore, the ambient experience content can be varied in energy levels, which is determined by features such as light intensity, sound intensity, level of distraction and more. It is also known that certain exam types have an optimal user state (especially cardiac scans require a certain BPM).

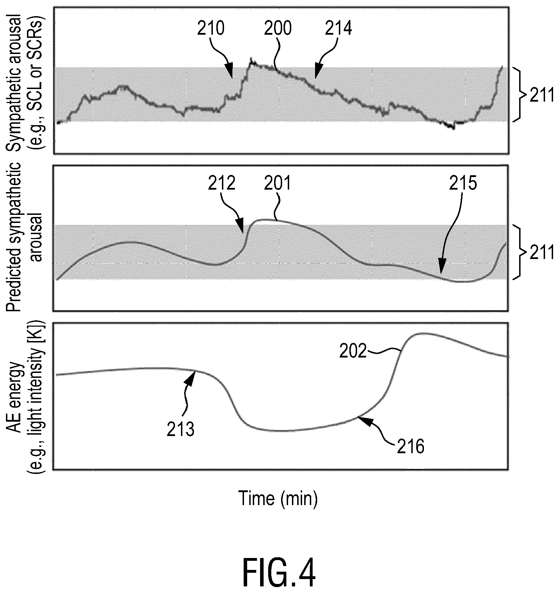

Main steps of this embodiment are illustrated in Fig. 4 showing diagrams of a sympathetic arousal signal 200 over time, a predicted sympathetic arousal signal 201 over time and an AE energy signal 202 over time.

Initially, based on the sympathetic arousal signal 200, a continuous trend detection is performed on the physiological trace, using e.g. known methods as e.g. described by Sittig, D. F., and Factor, M. (1990), Physiologic trend detection and artifact rejection: a parallel implementation of a multi-state Kalman filtering algorithm, Computer Methods and Programs in Biomedicine, 31(1), 1-10 and by Melek, W.W., et al., (2005), Comparison of Trend Detection Algorithms in the Analysis of Physiological Time-Series Data, IEEE transactions on bio-medical engineering, 52, 639-51, to detect an upward trend 210. Based on the detected trend (and the previous physiological trace 200) a predicted physiological arousal trace 201 is created, using e.g. a known method, such as Predictive Modeling - Time-Series Regression, Linear Regression Models - MATLAB & Simulink (mathworks.com)). Further, it is assessed whether this trace 201 stays within the optimal user state 211 for this scan type. If the predicted trace 201 will be higher than the optimal state 211 (detection point 212), calming features may be introduced at time point 213 into the ambient experience 202 (e.g., by lowering light intensity, using warmer light, playing soothing music, motivating meditative breathing, etc.).

Based on continuous trend detection on the physiological trace 201 a downward trend 214 may be detected. In this case, a predicted physiological arousal trace 201 is created in the same manner as explained above. If the predicted trace 201 will be lower than optimal state 211 (detection point 215), energizing features may be introduced at time point 216 into the ambient experience 202 (e.g., by providing brighter light, providing blue light, using upbeat music, using fast moving elements, etc.).

The input signal can generally be any signal that represents user state, for example, emotion detection in voice (albeit this signal is not continuous), other physiological signals such as heart rate variability, the hand grip of the user holding the button in the device, and/or pressure/movement on the mat. Any other adaptable ambient experience feature may be used to personalize this experience to the user state, for example, sound, specific content or storytelling, and/or temperature. Furthermore, the present invention may be applied to non-AE scans as long as there are means (e.g. a user interface and/or sound) near or within the bore that present content or is used as communication tool. For example, as calming feature a screen may say things like "try to relax and imagine yourself on a sunny beach".

According to another embodiment, the AE content may be controlled or updated based on user traits. The elements and information on user traits may include, e.g., exam card, personality assessment, self-reported needs, and a physiological trace acquired before the scan (retrieved with wearables or vital eye in the days before or in the waiting room). Depending on obtained user traits the user may need another type of content/AE guidance, for example, a distraction for anxious patients, an ambient experience for patients easily overstimulated (e.g., ADHD), and an energizing environment for tired or easily bored patients.

The AE content may further consider the scan protocol. Information on a specific imaging sequence that comes up is provided (e.g., use sequence information, how long will the sequence be, how loud will be it, etc.). Based on a predefined protocol the AE may automatically be adjust accordingly to match and steer the user state to fit the needs for the upcoming scan. In addition, the possibility for an automated feedback loop may be provided, e.g., by a patient input without a nurse or technologist interfering.

For example, in case the user wishes to change the AE environment to better suit its current user state and the scan procedure allows for this, the user can interact with the system to make this possible, e.g., by pressing a button, using speech recognition (volume up, next song, next theme), or using an eye-tracking menu enabling the user to choose items from a menu presented on an AE screen. This could be both based on direct content selection as well as providing the current user state as input to the system by answering basic questions whilst in the scanner. The user may e.g. be asked to indicate on a 5-point scale how relaxed he/she is, if he/she was able to follow the instructions, if he/she likes the current theme, etc., and then let the system adjust the content accordingly.

AE content may be used or adapted to achieve motion prevention. A prediction on when the user is going to move may be made, and information on how likely it is that the user will be in an undesired state during a particular sequence can be retrieved either from previous encounters with this user for the particular sequence, or from normative data bases, or both. In addition, the variability in a signal or trend can be detected (e.g. using a Kalman filter) using a sliding window. Based on the steepness of the slope it can be determined whether and what type of intervention is needed. When the prediction is that a user is going to move for a particular sequence/procedure during the imaging in the bore, a communication screen may be used, and the AE environment may select a predefined cognitive task, additional instructions, or ambient environment to distract or preventively instruct (e.g. using a message "please make sure to keep holding still") the user based upon the type of movement. Further, a predefined time window may be found to use for distraction. For instance, in one case the prediction may be that the user will move after three minutes, which in another case it may be five minutes. The cognitive task will then be different.

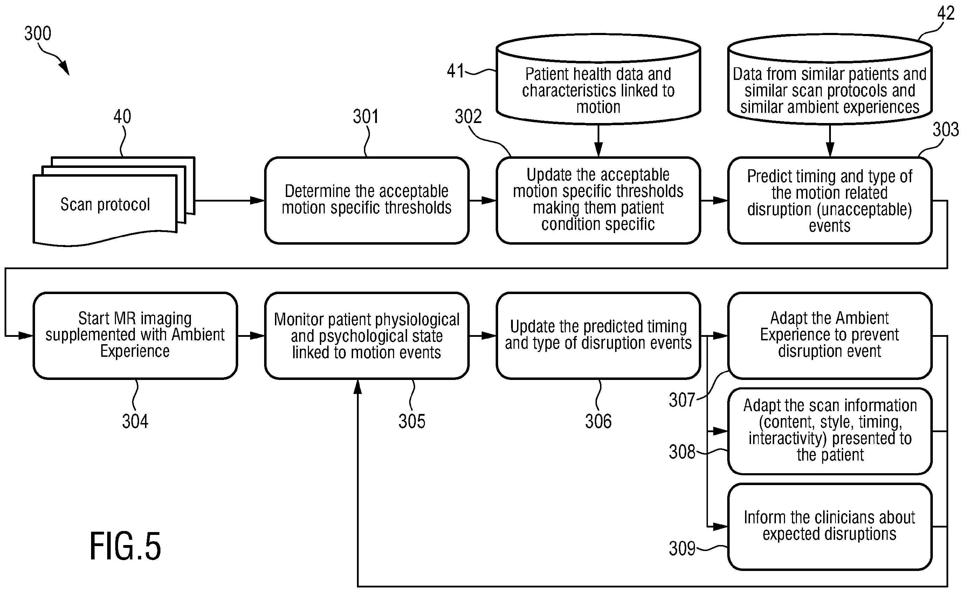

Fig. 5 shows a flowchart of another embodiment of an ambient experience control method 300 according to the present invention, which particularly focusses on motion prevention. In a first step 301, acceptable motion specific threshold(s) is (are) determined based on information on the scan protocol 40 (also called imaging protocol) to be used. This information includes information with regard to the predefined (minimum) imaging quality needed. This information can also be calculated based on existing images in an imaging (e.g. DICOM) database. For example, the signal to noise ratio or another image quality measure can be computed and its statistics (e.g., median and quantiles) can be used to determine the minimum acceptable threshold for the acceptable image quality.

In addition, if it is known (e.g. from the scan protocols 40) that a particular machine learning or artificial intelligence (AI) algorithm will be used to compensate for the potential motion artefacts, then the acceptable motion thresholds can be adapted taking into account the capabilities of such algorithm. Similarly, the capabilities (settings and known usages) of AI algorithms used by a radiologist while analysing the images can be also considered. Aspects related to the scanner (hardware) capabilities may be considered as well because the sensitivity of different hardware to motion artefacts is different.

Thus, in summary, the characteristics of software and hardware used for image acquisition, as well as the characteristics of the software used for an analysis of the images are used to calculate the acceptable thresholds for patient motion, without endangering the images becoming useless.

In a second step 302, the acceptable motion specific thresholds are updated to make them user condition specific, i.e. the thresholds determined in the previous step 301 are made user specific. Information on the user's health and other characteristics of the user linked to motion, as e.g. stored in a data base 41, may be used. For instance, if it is known that the user has a specific condition (e.g., the user moves when in a certain psychological/physiological state, certain drug has some motion related effect, the user moves after a period of inactivity, the user is likely to move a body part when in a certain position, etc.) the corresponding thresholds can be adapted. The adaptation in this case can be in terms of the threshold boundaries, for instance by lowering or increasing the thresholds. These adaptations may be with respect to the actions that are linked to likelihoods of thresholds being triggered. For example, the time window to start and stop the ambient experience or communication event can be determined in a user specific manner. In addition, the content of the AE and communication can be user specific. For instance, for some users the AE can be combined with the scan related information presented on the scanner monitors, while for some other users the technician involvement may be facilitated at the appropriate moments synched to the AE.

In a third step 303, timing and type of (unacceptable) disruption events related to motion are determined. Data from a similar user, similar scans, and/or similar ambient experiences as e.g. stored in a data base 42 may be used, wherein the classification of "similar" may depend on the hospital. Further, based on timing and type of (unacceptable) disruption events, the time and the type of motion may be predicted in step 304. Period of time may be specified either with respect to the start of the scan or with respect to another motion.

In step 304, the imaging procedure is started, supplemented with ambient experience.

In step 305, the user's physiological and psychological state linked to motion events is monitored.

In step 306, the predicted timing and the type of disruption events is updated. It can be specified, based upon the predefined protocol, how the update is performed and what type of update algorithm is used.

In step 307, the ambient experience is updated to prevent disruption events. For instance, the content can be adapted.

In step 308, the information about the upcoming scan sequence (e.g. content, style, timing, and/or interactivity) presented to the user is updated.

Finally, in step 309 the clinician is informed about expected disruptions.

The present invention may generally be applied with any medical imaging modalities, particularly where there is need for the user to be in a particular user state and avoid certain user states that may cause a disruption. Particular fields of application are MRI, CT and PET scans, where the user is place into a bore of the scanner and in which content may be presented to the user via a user interface, headphone, display, or other ambient experience unit(s).

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Ambient experience control device (30) for controlling the delivery of a personalized ambient experience to a user during a medical imaging scan, the device comprising:
- an input unit (31) configured to obtain
- scan information indicating scan-related information of a medical imaging scan including scan type information indicating the type of medical imaging scan and
- user information indicating user-related information including user state information indicating the current user state during the medical imaging scan;
- a processing unit (32) configured to
- predict the future user state based on the obtained scan information and the obtained user information,
- determine if the predicted future user state may cause a disruption of the medical imaging scan, and
- generate, if it has been determined that the predicted future user state may cause a disruption of the medical imaging scan, one or more ambient experience control signals configured to control one or more ambient experience units to generate or change a respective ambient experience provided by the respective ambient experience device; and
- an output unit (33) configured to output the one or more ambient experience control signals to the one or more ambient experience units.

2. Device according to claim 1,
wherein the processing unit (32) is configured to
- determine a trend of the user state over a period of time based on multiple data points of one or more pieces of information of the obtained scan information and the obtained user information and
- predict the future user state based on the determined trend.

3. Device according to any preceding claim,
wherein the processing unit (32) is configured to determine whether the predicted future user state is in line with a predetermined user state, in particular a predetermined user state for the type of medical imaging scan.

4. Device according to claim 3,
wherein the processing unit (32) is configured to determine whether the predicted future user state is in line with a predetermined user state by determining if the predicted future user state is below an upper state threshold or above a lower state threshold and/or if a distance metric between the predicted future user state and the predetermined user state is below a distance threshold.

5. Device according to any preceding claim,
wherein the processing unit (32) is configured to
- determine a disruption likelihood indicating the likelihood that the predicted future user state may cause a disruption of the medical imaging scan, and
- generate, if the determined disruption likelihood is above a likelihood threshold, one or more ambient experience control signals, in particular adapt the ambient experience control signals depending on the disruption likelihood and/or the time and/or duration of a disruption.

6. Device according to any preceding claim,
wherein the input unit (31) is configured
- to obtain, as scan information, information regarding one or more of type of medical imaging scan, length of medical imaging scan, type of medical imaging scanner, size of bore of the medical imaging scanner, sounds generated during medical imaging scan, and ideal user state during medical imaging scan; and/or
- to obtain, as user information, information regarding one or more of general user physiology, current user physiology, voice, movement, pressure, muscle activity, temperature, personality type, physiological trace before the medical imaging scan, health state, medication, scan history, problems during a prior medical imaging scan, needs reported by the user, frequency of problems during a medical imaging scan, eye movements, skin conductance level, sympathetic physiological arousal, stress level, pain level, electrodermal activity, sweating level, hand grip, respiration, pulse, heart rate variability, oxygen saturation, blood pressure, skin temperature, movement of one or more body parts, brain activity, user interaction information, user attention score, and game score.

7. Device according to any preceding claim,
wherein the processing unit (32) is configured to generate one or more ambient experience control signals configured to control one or more of
- intensity, duration and/or color of light or images,
- intensity, duration, frequency, content and/or type of sound or voice,
- ambient temperature and/or heating temperature,
- intensity, duration and/or type of a tactile stimulation, and
- intensity, duration and/or type of an odor emission.

8. Device according to any preceding claim,
wherein the input unit (31) is configured to receive user feedback during the medical imaging scan and
wherein the processing unit (32) is configured to use the received user feedback for generating the one or more ambient experience control signals.

9. Device according to any preceding claim,
wherein the processing unit (32) is configured to predict a time window when the user is going to move during the medical imaging scan based on one or more of
- movements of the user during a prior medical imaging scan;
- movements of the user during the medical imaging scan and/or during its preparation; and
- a database of typical movements and time windows of users as well as respective user information during a prior medical imaging scan.

10. Device according to any preceding claim,
wherein the processing unit (32) is configured to determine one or more of
- a general acceptable motion specific threshold indicating intensity, duration, type and frequency of motion that is generally acceptable and not regarded as causing a disruption of the medical imaging scan,
- a personalized acceptable motion specific threshold indicating intensity, duration, type and frequency of motion that is acceptable for the user and not regarded as causing a disruption of the medical imaging scan by the user,
- timing and/or type of motion-related disruption, and
- timing and/or type of motion of the user.

11. System (1) for proving a personalized ambient experience to a user (2) during a medical imaging scan, the system comprising:
- a medical imaging scanner (10) configured to perform a medical imaging scan of a user;
- one or more user state acquisition units (20) configured to acquire user state information indicating the current user state during the medical imaging scan;
- an ambient experience control device (30) according to any preceding claim for controlling the delivery of a personalized ambient experience to a user during the medical imaging scan; and
- one or more ambient experience units (40) configured to provide an ambient experience to the user according to one or more ambient experience control signals generated by the ambient experience control device.

12. System according to claim 11,
wherein the one or more user state acquisition units (20) comprise one or more of a user interface for inputting information, a retrieval unit for retrieving information from a database or record, a microphone, a movement detection unit, a pressure detection unit, a muscle activity detection unit, a temperature measurement unit, an eye tracking unit, a skin conductance measurement unit, a sympathetic physiological arousal detection unit, a stress level detection unit, a pain level detection unit, an electrodermal activity detection unit, a sweating level detection unit, a hand grip detection unit, a respiration sensor, a pulse sensor, a heart rate variability detection unit, an oxygen saturation sensor, and a blood pressure sensor, and/or
wherein the one or more ambient experience units (40) comprise one or more of a stimulation unit configured to stimulate one or more user senses, a stimulating environment combined with scan information, a stimulating environment where technician input and/or involvement is possible, a display, a lighting unit, a loudspeaker, an air condition, a body heating element and an odor dispenser.

13. System according to claim 11 or 12,
further comprising a user interface (50) allowing a user to provide user feedback during the medical imaging scan, in particular a button, voice analysis unit, or eye tracking unit.

14. Ambient experience control method for controlling the delivery of a personalized ambient experience to a user during a medical imaging scan, the method comprising:
- obtaining
- scan information indicating scan-related information of a medical imaging scan including scan type information indicating the type of medical imaging scan and
- user information indicating user-related information including user state information indicating the current user state during the medical imaging scan;
- predicting the future user state based on the obtained scan information and the obtained user information;
- determining if the predicted future user state may cause a disruption of the medical imaging scan;
- generating, if it has been determined that the predicted future user state may cause a disruption of the medical imaging scan, one or more ambient experience control signals configured to control one or more ambient experience units to generate or change a respective ambient experience provided by the respective ambient experience device; and
- outputting the one or more ambient experience control signals to the one or more ambient experience units.

15. Computer program comprising program code means for causing a computer to carry out the steps of the method as claimed in claim 14 when said computer program is carried out on the computer.
